# EUROPEAN PATENT SPECIFICATION

(11) **EP 1 511 510 B1**
(45) Date of publication and mention of the grant of the patent: **12.10.2005**
(21) Application number: 03732475.3
(22) Date of filing: 27.05.2003
(51) Int. Cl.: A61K 38/17, A61P 33/02

(54) **USE OF UK114 IN THE TREATMENT OF LEISHMANIASIS**
VERWENDUNG VON UK114 FÜR DIE BEHANDLUNG VON LEISHMANIASIS
UTILISATION DE LA PROTEINE UK114 DANS LE TRAITEMENT DE LA LEISHMANIOSE

(30) Priority: 04.06.2002 IT MI20021205
(43) Date of publication of application: 09.03.2005
(73) Proprietor: Pharmaproducts UK Limited, Merseyside L2 9TL (GB)
(72) Inventor: SALVAGGIO, Antonio, I-95021 Acicastello (CT) (IT); NICOLETTI, Ferdinando, I-95021 Cannizzaro (CT) (IT); MACRI', Battesimo, I-98168 Messina (IT)
(74) Representative: Minoja, Fabrizio
(86) International application number: PCT/EP2003/005551
(87) International publication number: WO 2003/101477

(56) References cited:
- WO-A-00/78329
- WO-A-98/42366
- WO-A-99/43340
- PANERAL A E ET AL: "CHRONIC ADMINISTRATION OF UK-114, A MULTIFUNCTIONAL EMERGING PROTEIN, MODULATES THE TH1/TH2 CYTOKINE PATTERN AND EXPERIMENTAL AUTOIMMUNE DISEASES" ANNALS OF THE NEW YORK ACADEMY OF SCIENCES, NEW YORK ACADEMY OF SCIENCES, NEW YORK, NY, US, vol. 876, 1999, pages 229-235, XP000971426 ISSN: 0077-8923
- NICOLETTI FERDINANDO ET AL: "Prevention and treatment of lethal murine endotoxemia by the novel immunomodulatory agent MFP-14." ANTIMICROBIAL AGENTS AND CHEMOTHERAPY, vol. 45, no. 5, May 2001 (2001-05), pages 1591-1594, XP002253826 ISSN: 0066-4804
- DATABASE BIOSIS [Online] BIOSCIENCES INFORMATION SERVICE, PHILADELPHIA, PA, US; July 2001 (2001-07) KHASKHELY NOOR MOHAMMAD ET AL: "Pre-exposure with low-dose UVA suppresses lesion development and enhances Th1 response in BALB/c mice infected with Leishmania (Leishmania) amazonensis." Database accession no. PREV200100345475 XP002253827 & JOURNAL OF DERMATOLOGICAL SCIENCE, vol. 26, no. 3, July 2001 (2001-07), pages 217-232, ISSN: 0923-1811

## Description

This invention relates to the use of the protein UK114, possibly associated with ubiquitin for the preparation of pharmaceutical or veterinary compositions to treat leishmaniasis in humans and animals.

The protozoa of the *Leishmania* genus are intracellular parasites of the macrophages and dendritic cells of the dog, man and numerous wild animals. On the basis of the classification criteria used in human medicine, leishmaniasis presents in three clinical forms: visceral (known in man as "kala-azar"), cutaneous and mucocutaneous.

In the case of the dog, a cutaneous and a visceral form were separately classified in the past because of the characteristic clinical picture, but they are now both regarded as progressive forms of the same disease, known as "generalised canine leishmaniasis".

The vector is a sand-fly of the *Phlebotomus* genus in the Old World and the *Lutzomyia* genus in the New World. The protozoa multiply in the sand-fly and are transformed into infectious organisms.

Parasites of the *Leishmania* genus appear as rounded or oval organisms in the macrophage, with the rod-like kinetoplast adjacent to the nucleus. The organism, which measures 2 to 5 µm in diameter, possesses a rudimentary flagellum that does not extend beyond the edge of the cell. This **amastigote** form of the parasite is ingested by the sand-fly during the blood meal. The protozoon is transformed in the intestine of the intermediate host into the **promastigote** form, characterised by a long free flagellum that protrudes from the anterior extremity of the parasite. The organism has an elongated shape and can grow to a length of 15 µm, excluding the flagellum, which usually has the same dimensions as the body.

The amastigotes ingested reach the intestine of the sand-fly, where they are transformed into promastigotes. The promastigotes divide repeatedly by binary fission, and subsequently migrate in the anterior direction. In the pharynx, the parasites turn into highly mobile metacyclic promastigotes, which migrate towards the proboscis. The promastigotes are transmitted to the new vertebrate host by means of the sand-fly's bite.

In the vertebrate host, the promastigotes are ingested by the monocytes/macrophages. After being ingested, the promastigote turns into an amastigote. The amastigotes divide by binary fission in the parasitophorous vacuole until their number is sufficient to rupture the macrophage. The amastigotes thus released are ingested by other macrophages.

The ability of the amastigotes to survive in the macrophages and spread throughout the body depends on factors intrinsic to the parasite and on factors associated with the type of cell-mediated immune response developed by the host. If parasitic macrophages are sufficiently stimulated by T-helper (Th) lymphocytes, they produce numerous lysosome enzymes and other factors including oxygen metabolites, hydrogen superoxide and peroxide and nitrous oxide (NO), which are toxic to the parasite.

The type of cell-mediated immune response and interleukin (IL) profile produced determine resistance or sensitivity to *Leishmania* infection. In laboratory animals, resistance to *Leishmania* infection is characterised by the **Th1** response, with production of IL-12 and interferon gamma (IFNγ) and activation of the macrophages which eliminate the parasite. Conversely, in animals sensitive to infection, the response is type **Th2**, characterised by production of IL-4 and IL-10 with consequent suppression of the parasiticidal activity of the macrophages and stimulation of the B lymphocytes with an increase in the production of immunoglobulins.

The humoral immune response in leishmaniasis is impressive, but not protective. The specific antibodies produced against *Leishmania* have no neutralising action against the parasite.

In animals sensitive to the disease the protozoon spreads throughout the body, in the macrophages. The parasite has been observed in all the organs and tissues of the body except the central nervous system. Slow, continuous contact between the parasitic antigen and the immunocompetent cells forms the basis for the pathogenetic development of the disease, which is characterised by:
hyperglobulinaemia, generally polyclonal, associated with continual stimulation of the B lymphocytes, which causes an increase in total proteins and inversion of the albumin/globulin ratio;
production of auto-antibodies, probably due to a cross-reaction between parasitic antigens and self-antigens, causing thrombocytopenia and anaemia;
production and deposit of immunocomplexes responsible for the vasculitis, glomerulonephritis and polyarthritis syndromes.

The pathogenesis of the skin lesions present in most sufferers is not yet clear. According to some authors, the persistence of the parasite in the macrophage continually stimulates infiltration by inflammatory cells, especially plasma cells, macrophages and lymphocytes, into the dermis. According to other authors, the deposit of immunocomplexes is the main cause of dermatitis which, on histological examination, often presents lesions similar to those caused by other diseases induced by immunocomplexes, such as systemic Lupus erythematosus. Finally, the skin alterations may be the result of vasculitis.

The symptoms of canine leishmaniasis are highly variable, and may include peripheral lymphadenopathy (over 90% of infected subjects), skin lesions (>80%), chronic conjunctivitis (50%), onychogryphosis (40%), anorexia (>35%), increased appetite (30%), weight loss (30%), fever (20%), kidney failure (20%), epistaxis (10%), uveitis (8%) and gait disorders (6%).

The skin signs are among the most important in the disease. Various types of macroscopic and microscopic lesions have been described in canine leishmaniasis: dry exfoliative dermatitis, ulcerative dermatitis, nodular dermatitis, sterile pustular dermatitis, paronychia, and nasal and/or digital hyperkeratosis. The skin lesions are generally chronic, symmetrical and not itchy.

Recent studies clearly demonstrate the existence of a TNF-independent compensation mechanism able to activate the macrophages in the anti-leishmania response. As the **Th1** response mediated by interleukin-12 (IL-12) and interferon gamma (IFNγ) is paradoxically responsible not only for activation of the macrophages, but also for nearly all the symptoms of leishmaniasis, it may be advantageous to boost this compensation mechanism by inhibiting the **Th1** response.

The current elective treatment, based on antimony gluconate administered by infiltration (in cutaneous 1.) or injection (in the other forms) can cause toxic effects (nausea and vomiting) sufficiently serious to require discontinuance of the treatment, which is replaced by treatment with aromatic diamines such as pentamidine, whose tolerability is generally poor.

It has now been found that the protein with molecular weight 14 kDa in SDS-PAGE, obtainable by extraction from mammal liver with perchloric acid, called **UK114** and disclosed in EP 574394 and US 5792744, is useful in the treatment of leishmaniasis, possibly associated with ubiquitin (**UK110**).

Recombinant protein UK114 is known from WO 00/63368.

Paneral A. E. et al. (Annals Of The New York Academy Of Sciences, New York Academy Of Sciences, New York, NY, US, vol. 876, 1999, pages 229-235) discovered that the use of UK114 as an immunomodulator induced a shift towards a Th2 cytokine profile (increasing IL-4 and downregulating IFN-gamma, IL-2 and TNF-alpha). UK114 was used to treat adjuvant-induced arthritis and diabetes, both characterised through a Th1 cytokine profile. Shifting the Th1 to Th2 pattern delayed the development of symptoms and reduced severity. The aspect of reducing TNF-alpha production was found to be interesting in the light of other autoimmune and inflammatory diseases.

Khaskhely Noor Mohammad et al. (Journal Of Dermatological Science, vol. 26, no. 3, July 2001 (2001-07), pages 217-232), found that pre-exposure with low-dose UVA suppresses lesion development and induces a response switch from Th2 to Th1 pattern (upregulation of IFN-gamma, TNF-alpha; downregulation of IL-4 and IL-10) in BALB/c mice infected with Leishmania amazonensis.

Subcutaneous administration of **UK114** and ubiquitin to a group of 10 dogs with manifest clinical symptoms of leishmaniasis (peripheral lymphadenopathy and skin lesions of a high degree, mainly represented by sores and bleeding ulcers with loss of substance, anorexia and weight loss), at the doses and times indicated in the table, led to complete healing of all the lesions during the treatment period.

No adverse effects were observed during the treatment.

These findings demonstrate that the administration of **UK114** and ubiquitin cures the clinical symptoms of leishmaniasis in a totally safe manner. This is very interesting in view of the high toxicity of the drugs currently used in treatment, and the possibility of a response that is sometimes unsatisfactory from the clinical standpoint.

**TABLE**

| | | |
|---|---|---|
| Patient's weight < 10 kg | **1mg/day** subcutaneously for **6** days 7th day: rest | |
| | **1 mg/day** subcutaneously for **6** more days | |
| Patient's weight > 10 kg | As above, but doubling the dose: **2 mg/day** | |

According to the invention, protein UK114 of extractive or recombinant origin is therefore to be opportunely administered to subjects suffering from leishmaniasis by the parenteral route, in particular subcutaneously or intramuscularly, at doses ranging between 0.5 and 10 mg a day, until the disappearance or substantial reduction of the symptoms. The compositions according to the invention, in the form of solutions or suspensions in preferably aqueous sterile solvents, may also contain ubiquitin in a quantity corresponding to 0.1-5 mg per unit dose.

### BIBLIOGRAPHY

1. **Urquhart G.M., Armour J., Duncan J.L., Dunn A.M, Jennings F.W.** Veterinary Parasitology 2^{nd} ed., Italian edition edited by C. Genchi, UTET 1998.
2. **Murray H.W., Jungbluth A., Ritter E., Montelibano C., Marino M.W.** Visceral Leishmaniasis in Mice Devoid of Tumor Necrosis Factor and Response to Treatment, Infection and Immunity, November 2000, p. 6289-6293, Vol. 68, No. 11.
**3. Katzung B.G.** Basic & Clinical Pharmacology, 1995 by Appleton & Lange, E.N., Connecticut.

## Claims

1. Use of the protein UK114, possibly in combination with ubiquitin, for the preparation of pharmaceutical or veterinary compositions for the treatment of leishmaniasis in humans and animals.

## Patentansprüche

1. Verwendung des Proteins UK114, gegebenenfalls in Kombination mit Ubiquitin zur Herstellung von pharmazeutischen oder veterinärmedizinischen Erzeugnissen für die Behandlung von Leishmaniasis bei Menschen und Tieren.

## Revendications

1. Utilisation de la protéine UK114, éventuellement en combinaison avec l'ubiquitine, pour la préparation de compositions pharmaceutiques ou vétérinaires pour le traitement de la leishmaniose chez les humains et les animaux.
